# EUROPEAN PATENT APPLICATION

(11) **EP 4 015 601 A1**
(43) Date of publication of application: **22.06.2022**
(21) Application number: 20215789.7
(22) Date of filing: 18.12.2020
(51) Int. Cl.: C10G 2/00, B01J 23/889, C07C 29/156

(54) **FISCHER-TROPSCH PROCESSES WITH MODIFIED PRODUCT SELECTIVITY**

(71) Applicant: BP P.L.C., London SW1Y 4PD (GB)
(72) Inventor: The designation of the inventor has not yet been filed
(74) Representative: Mathys & Squire

(57) **Abstract**

The present disclosure relates generally to compositions and processes for modifying Fischer-Tropsch product selectivity. In particular, the disclosure provides for a for converting a mixture of hydrogen and carbon monoxide gases to a product composition comprising alcohols and liquid hydrocarbons via Fischer-Tropsch synthesis in the presence of a supported cobalt-manganese Fischer-Tropsch synthesis catalyst, the process comprising: contacting the catalyst with a first gaseous feed comprising carbon monoxide and hydrogen for at least 12 hours to provide via Fischer-Tropsch synthesis a first product composition comprising C₅₊ hydrocarbons and alcohol; then contacting the catalyst with a first selectivity gaseous composition comprising at least 35 vol% H₂ and a H₂:CO molar ratio of at least 2; and then contacting the catalyst with a second gaseous feed comprising carbon monoxide and hydrogen to provide a second product composition comprising C₅₊ hydrocarbons, with a selectivity of no more than 5% for alcohols. Optionally, the catalyst selectivity to alcohols can be reversed by contacting the catalyst with a second selectivity gaseous composition comprising CO or a H₂:CO molar ratio of at below 1.5.

## Description

### Field

The present disclosure relates to Fischer-Tropsch processes with a switchable product selectivity formed from a mixture of hydrogen and carbon monoxide gases.

### Technical Background

The conversion of synthesis gas into hydrocarbons by the Fischer-Tropsch process has been known for many years. The growing importance of alternative energy sources has resulted in renewed interest in the Fischer-Tropsch (FT) process as it allows a direct and environmentally acceptable route to high-quality fuels and feedstock chemicals through use of bio-derived carbon sources.

FT processes are known to produce linear hydrocarbons for use in fuels, as well as oxygenates which serve as valuable feedstock chemicals. The hydrocarbon fuel deriving from FT processes is better able to meet increasingly stringent environmental regulations compared with conventional refinery-produced fuels, as FT-derived fuels typically have lower contents of sulfur, nitrogen, and aromatic compounds which contribute to the emission of potent pollutants such as SO₂, NOₓ, and particulates. Alcohols derived from FT processes often have a higher octane rating than hydrocarbons and thus burn more completely, thereby reducing the environmental impact of such a fuel. Alcohols and other oxygenates obtained may also be used as reagents in other processes, such as in the synthesis of lubricants.

A variety of transition metals have been identified to be catalytically active in the conversion of synthesis gas into hydrocarbons and oxygenated derivatives thereof. In particular, cobalt, nickel, and iron have been studied, typically in combination with a support material, of which the most common are alumina, silica and carbon.

Typically, the principal focus in producing Fischer-Tropsch synthesis catalysts is on improving activity and selectivity for C₅₊ hydrocarbons (e.g., paraffins). While they are industrially-important products in their own right, alcohols are typically produced merely as a side product of Fischer-Tropsch processes, in much lower yield. Typically, Fischer-Tropsch processes are designed around particular catalysts which give certain product distributions. Modifying a reactor to have different selectivity for certain products involves shutting down the reactor, physically removing the catalyst, and installing a new catalyst. This process is costly, both in terms of catalyst and in idle reactor time.

Accordingly, there exists a need to improve the activity and selectivity of Fischer-Tropsch processes, especially with regard to control over process selectivity for particular products.

### SUMMARY

The inventors have found a process to switch the selectivity of certain Fischer-Tropsch processes between higher and lower selectivities for the production of alcohol products and other oxygenates. Advantageously, the switching process is found to be reversible, allowing facile control over reaction products. Moreover, the present inventors have determined catalyst treatments that can reduce alcohol selectivity even for catalysts having already-low alcohol selectivities.

Accordingly, one aspect of the disclosure provides for a process for converting a mixture of hydrogen and carbon monoxide gases to a product composition comprising alcohols and liquid hydrocarbons via Fischer-Tropsch synthesis in the presence of a supported cobalt-manganese Fischer-Tropsch synthesis catalyst, the process comprising:
optionally, contacting the catalyst with a first gaseous feed comprising carbon monoxide and hydrogen for at least 12 hours to provide via Fischer-Tropsch synthesis a first product composition comprising C₅₊ hydrocarbons and one or more alcohols with a first selectivity for alcohols and a first selectivity for C5+ hydrocarbons; then
contacting the catalyst with a first selectivity-modifying gaseous composition comprising at least 35 vol% H₂ and a H₂:CO molar ratio of at least 2 at a pressure in the range of 20 barg to 40 barg and a temperature in the range of 150 °C to 300 °C; and then
contacting the catalyst with a second gaseous feed comprising carbon monoxide and hydrogen to provide a second product composition comprising C₅₊ hydrocarbons, with a selectivity of no more than 5% for alcohols, and/or a selectivity of at least 80% for C₅₊ hydrocarbons.

Another aspect of the disclosure provides a process as otherwise described herein, the process further comprising: monitoring the second reaction product selectivity for alcohols and/or C₅₊ hydrocarbons; determining if the alcohols selectivity is greater than an alcohols threshold value, and/or if the C₅₊ hydrocarbons selectivity is less than a hydrocarbons threshold value; and if the alcohol selectivity is greater than the alcohols threshold value, and/or if the C₅₊ hydrocarbons selectivity is less than the hydrocarbons threshold value, contacting the catalyst with the first selectivity-modifying gaseous composition.

Another aspect of the disclosure provides a process as otherwise described herein, the process further comprising, after contacting the catalyst with the second gaseous feed:
contacting the catalyst with a second selectivity-modifying gaseous composition comprising the range of pure carbon monoxide through to a H₂ and CO in a ratio in 1.8:1 at a pressure in the range of 3 barg to 50 barg and at a temperature in the range of 100 °C and 300 °C; and then
contacting the catalyst with a third gaseous feed comprising carbon monoxide and hydrogen to provide a third product composition comprising C₅₊ hydrocarbons and alcohol, with a selectivity of greater than 5% for alcohols, and/or a selectivity of no more than 95% for C₅₊ hydrocarbons.

Other aspects of the disclosure will be apparent to those skilled in the art in view of the description that follows.

### DETAILED DESCRIPTION

The present disclosure is concerned with processes to modify alcohol and hydrocarbon selectivity in a Fischer-Tropsch process. As described in International Patent Application Publication no. 2019/154885 and which is hereby incorporated herein by reference in its entirety, the use of catalysts including manganese can provide somewhat increased amounts of alcohol in the product stream. In certain embodiments, increased amounts of alcohol can be desirable, as they can be separated as valuable products in their own right. The inventors have now found that treating a used Fischer-Tropsch catalyst with a first selectivity-modifying gaseous composition comprising hydrogen under certain conditions can at least temporarily reduce this alcohol selectivity, allowing for the synthesis of relatively more hydrocarbon . Advantageously, subsequent treatment with a second selectivity-modifying gaseous mixture under certain conditions results in an increase in alcohol selectivity. Accordingly, such processes allow flexible determination of product selectivity within the same reactor using the same catalyst and even the same CO/H₂ feed.

Moreover, the present inventors have determined that certain selectivity-modifying treatments described herein can be used to further reduce alcohol selectivity of catalysts having already-low alcohol selectivities .

Accordingly, one aspect of the disclosure provides for a process for converting a mixture of hydrogen and carbon monoxide gases to a product composition comprising alcohols and liquid hydrocarbons via Fischer-Tropsch synthesis in the presence of a supported cobalt-manganese Fischer-Tropsch synthesis catalyst, the process comprising:
optionally, contacting the catalyst with a first gaseous feed comprising carbon monoxide and hydrogen for at least 12 hours to provide via Fischer-Tropsch synthesis a first product composition comprising C₅₊ hydrocarbons and one or more alcohols with a first selectivity for alcohols and a first selectivity for C5+ hydrocarbons; then
contacting the catalyst with a first selectivity-modifying gaseous composition comprising at least 35 vol% H₂ and a H₂:CO molar ratio of at least 2 at a pressure in the range of 20 barg to 40 barg and a temperature in the range of 150 °C to 300 °C; and then
contacting the catalyst with a second gaseous feed comprising carbon monoxide and hydrogen to provide a second product composition comprising C₅₊ hydrocarbons, with a selectivity of no more than 5% for alcohols, and/or a selectivity of at least 80% for C₅₊ hydrocarbons.

The term "hydrocarbons" is used herein to signify carbon- and hydrogen-containing compounds (e.g., alkanes and alkene) without oxygen- or nitrogen-containing functional groups. Accordingly, "hydrocarbons" are devoid of any hydroxy, aldehyde, ketone, ether, ester, or carboxylic acid functional group. Compounds that do contain one or more hydroxy, aldehyde, ketone, ether, ester, or carboxylic acid functional groups are referred to herein as "oxygenates." An alcohol is a type of oxygenate.

The term "liquid hydrocarbons" used herein in reference to the products of the Fischer-Tropsch reaction refers to C₄ to C₂₄ hydrocarbons. In certain embodiments as otherwise described herein, the liquid hydrocarbons are predominantly linear hydrocarbons, e.g., at least 50 wt%, at least 75 wt%, or even at least 90 wt% linear hydrocarbons.

The term "alcohol" as used herein in reference to the products of the Fischer-Tropsch reaction refers to an alcohol having any number of carbon atoms. For example, in certain embodiments the alcohols of the Fischer-Tropsch product have from one to 30 carbons. The alcohols are typically acyclic and may be straight- or branched-chain, preferably straight-chain. In certain embodiments as otherwise described herein, the alcohols comprise at least 50 wt% linear alpha alcohols, such as at least 70 wt% linear alpha alcohols or at least 80 wt% linear alpha alcohols.

In certain embodiments as otherwise described, the alcohols prepared by the process of the present disclosure include a major proportion (at least 40 wt%) of short- and medium-chain length C₁ to C₂₄ alcohols, for example, at least 50 wt% C₁ to C₈ alcohols or even at least 60 wt% C₁ to C₂₄ alcohols. But in other embodiments, the alcohols prepared by the process of the present disclosure include a major proportion (above 50 wt%) long-chain length C₉ to C₂₅ alcohols. The amount of alcohols produced by the Fischer-Tropsch reaction, and the relative proportion of particular alcohols produced, is determined by GC or GC mass spectrometry.

In certain embodiments as otherwise described herein, the contacting of the catalyst with the first gaseous feed is performed. In such cases, this first process step can be performed, e.g., at a relatively higher alcohol selectivity, to form a relatively higher proportion of alcohol product. For example, in certain such embodiments, the contacting with the first gaseous feed is performed with a first selectivity for alcohols of greater than 5%, e.g., at least 7%. In certain embodiments, the contacting with the first gaseous feed is performed with a first selectivity for alcohols of at least 10%, e.g., at least 12%.

In certain embodiments, the catalyst may require activation before being used to make desired product streams. Accordingly, in certain embodiments, the process further comprises, before contacting the catalyst with the first gaseous feed and/or the first selectivity-modifying composition, contacting the catalyst with an activation gaseous composition comprising at least 50 vol% H₂ at a pressure in the range of 2 barg to 30 barg and a temperature in the range of 250 °C to 450 °C. In particular embodiments, the process further comprises contacting the catalyst with an activation gaseous composition comprising at least 35 vol% H₂ at a pressure in the range of 6 barg to 20 barg (e.g., 10 to 15 barg) and a temperature in the range of 275 °C to 400 °C (e.g., 280 °C to 350 °C).

The contact of the catalyst with the first selectivity-modifying gaseous composition is used to alter the selectivity of process. Notably, the present inventors have determined that this process step, performed as described herein, can reduce selectivity for alcohols and increase selectivity for hydrocarbons. Without wishing to be bound by theory, it is presently believed that the first selectivity-modifying gaseous composition functions to chemically alter the catalyst by changing catalyst identity or morphology to change the product distribution produced. In one aspect of the disclosure, the first selectivity-modifying gaseous composition comprises at least 35 vol% H2 and has an H2:CO molar ratio of at least 2. In certain embodiments as otherwise described herein, the first selectivity gaseous composition comprises at least 40 vol% H₂, or at least 50 vol% H₂. For example, in certain embodiments, the first selectivity gaseous composition comprises at least 60 vol% H₂, or at least 70 vol% H₂, or at least 80 vol% H₂ (e.g., at least 90 vol% H₂, or at least 95 vol% H₂, or at least 99 vol% H₂, or substantially pure H₂). In certain embodiments as otherwise described herein, the first selectivity gaseous composition has an H₂:CO molar ratio of at least 2, or at least 3. In certain embodiments, the molar ratio is at least 4, or at least 5 (e.g., at least 7, or at least 8, or at least 10, or at least 20). Carbon monoxide need not be present in the first selectivity-modifying gaseous composition, and so in particular embodiments, the first selectivity gas comprises substantially no carbon monoxide.

The contacting the catalyst with the first selectivity-modifying gaseous composition is conducted at a pressure and temperature and for a time sufficient to effect a desired change in product selectivity. In one aspect of the disclosure, the pressure is in the range of 20 barg to 40 barg and the temperature in the range of 150 °C to 300 °C. In certain embodiments as otherwise described herein, the contacting of the catalyst with the first selectivity-modifying gaseous composition is done at a pressure in the range of 15 barg to 35 barg (e.g., in the range of 20 barg to 32 barg, or at approximately 30 barg). In certain embodiments as otherwise described herein, the contacting of the catalyst with the first selectivity-modifying gaseous composition is done at a temperature in the range of 150 °C to 300 °C (e.g., in the range of 200 °C to 250 °C). In certain embodiments as otherwise described herein, the contacting of the catalyst with the first selectivity-modifying gaseous composition is performed for a time up to 48hrs (e.g. in the range of 1 to 24hrs) .

As described above, after the catalyst is contacted with the first selectivity-modifying gaseous composition, it can be contacted with a second gaseous feed comprising carbon monoxide and hydrogen. In certain embodiments, the second gaseous feed is substantially identical to the first gaseous feed. But in other embodiments, it can be different. The contacting with the second gaseous feed can be performed to provide a second product composition including C5+ hydrocarbons, with a second selectivity for alcohols of no more than 5%, and/or a second selectivity for C₅₊ hydrocarbons of at least 80%.

In certain embodiments as otherwise described herein, the contacting the catalyst with the second gaseous feed is performed for at least 6 hours, or at least 12 hours to provide the second product composition. For example, the contacting the catalyst with the second gaseous feed may be performed for at least 1 day, or at least 2 days, or at least 7 days. There is no upper limit on the amount of time the contacting the catalyst with the second gaseous feed is performed, other than the general limit for Fischer-Tropsch processes due to catalyst degradation, etc. The contacting may continue until it is determined that sufficient amounts of the second product composition have been produced, or maintenance must be performed. Alternatively, the reaction may be discontinued, and the reactor used for other means.

The second product composition has an advantageously low selectivity for alcohols. Accordingly, in certain embodiments as otherwise described herein, the selectivity of the second product composition for alcohols (i.e., for C₁-C₂₄ alcohols, e.g., for C₁-C₈ alcohols) is no more than 5%, for example, no more than 4%, or no more than 3%. In certain embodiments as otherwise described herein, the selectivity of the second product composition for alcohols (e.g., for C₁-C₂₄ alcohols, or for C₁-C₈ alcohols) is no more than 2%, for example, no more than 1%, or no more than 0.8%. As used herein, "selectivity" for a given component is measured as the molar fraction of carbon monoxide that is reacted in the process (i.e., not including unreacted carbon monoxide) that is converted to that product.

In certain embodiments as otherwise described in which the process step making the first product composition is included, the second selectivity for alcohols (i.e., of the second product composition) is less than the first selectivity for alcohols (i.e., of the first product composition). For example, in certain embodiments as otherwise described herein, the second selectivity for alcohols is no more than 75% of the first selectivity for alcohols (e.g., no more than 60%, or no more than 50%). In certain embodiments as otherwise described herein, the second selectivity for alcohols is no more than 40% of the first selectivity for alcohols (e.g., no more than 30%, or no more than 25%). In some embodiments as otherwise described herein, the second selectivity for alcohols is no more than 20% of the first selectivity for alcohols (e.g., no more than 15%, or no more than 10%). For the sake of clarity, if the first selectivity for alcohols is 14%, and the second selectivity for alcohols is 2.8%, the second selectivity for alcohols is 20% of the first selectivity for alcohols.

In certain embodiments as otherwise described herein, the contacting with the first selectivity-modifying gaseous composition reduces the second selectivity for alcohols (i.e., of the second product composition) such that it is less than a reference selectivity for alcohols of the catalyst before the contacting with the first selectivity-modifying gaseous composition for the same feed and reaction conditions used to make the second product composition. For example, in certain embodiments as otherwise described herein, the second selectivity for alcohols is no more than 75% of the reference selectivity for alcohols (e.g., no more than 60%, or no more than 50%). In certain embodiments as otherwise described herein, the second selectivity for alcohols is no more than 40% of the reference selectivity for alcohols (e.g., no more than 30%, or no more than 25%). In some embodiments as otherwise described herein, the second selectivity for alcohols is no more than 20% of the reference selectivity for alcohols (e.g., no more than 15%, or no more than 10%).

In certain embodiments, the second product composition advantageously has a high selectivity for C₅₊ hydrocarbons. Accordingly, in certain embodiments as otherwise described herein, the second selectivity (i.e., of the second product composition) for C₅₊ hydrocarbons is at least 75%, e.g., at least 80%. For example, in certain embodiments, the second selectivity for C₅₊ hydrocarbons is e.g., least 85%, or at least 90%.

In certain embodiments as otherwise described herein, the second selectivity (i.e., of the second product composition) for C₅₊ hydrocarbons is greater than the first selectivity (i.e., of the first product composition) for C₅₊ hydrocarbons. For example, in certain embodiments, the second selectivity for C₅₊ hydrocarbons is at least 105%, e.g., at least 110%, of the first selectivity for C₅₊ hydrocarbons. In particular embodiments, the second selectivity for C₅₊ hydrocarbons is at least 115%, e.g., at least 120% or at least 125%, of the first selectivity for C₅₊ hydrocarbons.

In certain embodiments as otherwise described herein, the second selectivity (i.e., of the second product composition) for C₅₊ hydrocarbons is greater than a reference selectivity for C₅₊ hydrocarbons of the catalyst before the contacting with the first selectivity-modifying gaseous composition for the same feed and reaction conditions used to make the second product composition. For example, in certain embodiments, the second selectivity for C₅₊ hydrocarbons is at least 105%, e.g., at least 110%, of the reference selectivity for C₅₊ hydrocarbons. In particular embodiments, the second selectivity for C₅₊ hydrocarbons is at least 115%, e.g., at least 120% or at least 125%, of the reference selectivity for C₅₊ hydrocarbons.

An advantage of the processes of the present disclosure is that they may be used to alter the product selectivity of a Fischer-Tropsch reaction through a convenient treatment of the catalyst. Accordingly, in certain embodiments as otherwise described herein, the contacting the catalyst with the first gaseous feed, the contacting the catalyst with the first selectivity-modifying gaseous composition, and the contacting the catalyst with the second gaseous feed are performed in a reactor without removing the catalyst therefrom.

The contacting of the catalyst with the second gaseous feed (i.e., after the treatment with the first selectivity-modifying gaseous composition) to provide the second product composition can be performed under a variety of Fischer-Tropsch reaction conditions. In certain embodiments as otherwise described here, the contacting the catalyst with the second gaseous feed occurs at a pressure in the range of 150 °C to 300 °C (e.g., in the range of 175 °C to 275 °C, or in the range of 200 °C to 250 °C). In certain embodiments as otherwise described here, the contacting the catalyst with the second gaseous feed occurs at a pressure of 10 barg to 100 barg, or 20 barg to 80 barg. Of course, the person of ordinary skill in the art will appreciate that other conditions can be used. The conditions can be similar to those used to make the first product composition, when

As described above, the first selectivity-modifying gaseous composition functions to modify the selectivity of the catalyst to reduce the selectivity for alcohols. It may occur, under certain reaction conditions, that the selectivity of the second product composition may drift over time to provide relatively more alcohol product. Repeated treatment with the first selectivity-modifying gaseous composition may be used to restore the selectivity as desired. Accordingly, in certain embodiments, the catalyst is contacted with the first selectivity-modifying gaseous composition a plurality of times, with contact with the second feed composition to form second product composition after each.

Monitoring can be used to help maintain the process in a low-alcohol product state. In certain embodiments, a process as otherwise described herein further comprises: monitoring the second selectivity for alcohols and/or C₅₊ hydrocarbons; determining whether the second selectivity for alcohols is greater than an alcohols threshold value, and/or whether the second selectivity for C₅₊ hydrocarbons is less than a hydrocarbons threshold value; and, if the second selectivity for alcohols is greater than the alcohols threshold value, and/or if the second selectivity for C₅₊ hydrocarbons is less than the hydrocarbons threshold value, contacting the catalyst with the first selectivity gaseous composition, e.g., as described above. The person of ordinary skill in the art will select an alcohol threshold value depending on the desired product composition. For example, in certain embodiments, the alcohols threshold value is no more than 10% (e.g., no more than 8%, or no more than 6%, or no more than 5%, or no more than 4%, or no more than 3%). In certain embodiments as otherwise described herein, the hydrocarbons threshold value is at least 85%, e.g., at least 90%. The alcohols threshold value can also be defined with respect to the second selectivity for alcohol, for example, at a value that is no more than 150% of the second selectivity for alcohols, e.g., a value no more than 125% of the second selectivity for alcohols, or no more than 110% of the second selectivity for alcohols. Similarly, the hydrocarbons threshold value can be defined with respect to the second selectivity for C₅₊ hydrocarbons, e.g., at a value of at least 70% of the second selectivity for C₅₊ hydrocarbons, e.g., a value at least 80%, or at least 90% of the second selectivity for C₅₊ hydrocarbons.

It has also been found that the selectivity for alcohols can be increased after contacting the catalyst with the second gaseous feed by treatment with a second selectivity gaseous composition. Advantageously, this allows modification of the Fischer-Tropsch reaction selectivity to switch back to relatively more alcohols according to specific needs, and may be performed in the same reactor without removing the catalyst or otherwise mechanically altering the reaction zone. Accordingly, in certain embodiments as otherwise described herein, the process further comprises, after contacting the catalyst with the second gaseous feed: contacting the catalyst with a second selectivity gaseous composition ranging from pure carbon monoxide through to a H₂ and CO in a ratio in the range up to 1.5:1 at a pressure in the range of 5 barg to 40 barg and at a temperature in the range of 100 °C and 300 °C; and then contacting the catalyst with a third gaseous feed comprising carbon monoxide and hydrogen to provide a third product composition comprising C₅₊ hydrocarbons and alcohol, with a third selectivity for alcohols of at least 5%, and/or a third selectivity for C₅₊ hydrocarbons of no more than 92%.

In certain embodiments as otherwise described herein, the contacting the catalyst with the third gaseous feed is performed for at least 6 hours, or at least 12 hours to provide a third product composition. For example, the contacting the catalyst with the third gaseous feed may be performed for at least 1 day, or at least 2 days, or at least 7 days. There is no upper limit on the amount of time the contacting the catalyst with the third gaseous feed is performed, other than the typical limitations of Fischer-Tropsch processes. The contacting may continue until it is determined that sufficient amounts of the third product composition have been produced, or maintenance must be performed. Alternatively, the reaction may be discontinued and the reactor used for other means.

Accordingly, one aspect of the disclosure provides for a process for converting a mixture of hydrogen and carbon monoxide gases to a product composition comprising alcohols and liquid hydrocarbons via Fischer-Tropsch synthesis in the presence of a supported cobalt-manganese Fischer-Tropsch synthesis catalyst, the process comprising:optionally, contacting the catalyst with a first gaseous feed comprising carbon monoxide and hydrogen for at least 12 hours to provide via Fischer-Tropsch synthesis a first product composition comprising C₅₊ hydrocarbons and one or more alcohols with a first selectivity for alcohols and a first selectivity for C5+ hydrocarbons; then
contacting the catalyst with a selectivity-modifying gaseous composition comprising H₂ and CO in a ratio in the range of pure carbon monoxide to a H2:CO ratio of 1.5:1 at a pressure in the range of 5 barg to 40 barg and at a temperature in the range of 100 °C and 300 °C; and then
contacting the catalyst with a second gaseous feed comprising carbon monoxide and hydrogen to provide a third product composition comprising C₅₊ hydrocarbons and alcohol, with a selectivity of greater than 5% for alcohols, and/or a selectivity of no more than 92% for C₅₊ hydrocarbons.

The third product composition has an advantageously higher selectivity for alcohols. Accordingly, in certain embodiments as otherwise described herein, the selectivity of the third product composition for alcohols (i.e., for C₁-C₂₄ alcohols, e.g., for C₁-C₈ alcohols) is greater than 5%, e.g., at least 7%. In certain embodiments as otherwise described herein, the third selectivity of the third product composition for alcohols (e.g., for C₁-C₂₄ alcohols, or for C₁-C₈ alcohols) is at least 10%, e.g., at least 12%.

The third product composition has an advantageously higher selectivity for alcohols compared to the second product composition. In certain embodiments as otherwise described in, the second selectivity for alcohols (i.e., of the second product composition) is less than the third selectivity for alcohols (i.e., of the third product composition). For example, in certain embodiments as otherwise described herein, the second selectivity for alcohols is no more than 75% of the third selectivity for alcohols (e.g., no more than 60%, or no more than 50%). In certain embodiments as otherwise described herein, the second selectivity for alcohols is no more than 40% of the third selectivity for alcohols (e.g., no more than 30%, or no more than 25%). In some embodiments as otherwise described herein, the second selectivity for alcohols is no more than 20% of the third selectivity for alcohols (e.g., no more than 15%, or no more than 10%).

In certain embodiments as otherwise described herein, the contacting with the second selectivity-modifying gaseous composition increases the third selectivity for alcohols (i.e., of the third product composition) such that it is more than a reference selectivity for alcohols of the catalyst before the contacting with the second selectivity-modifying gaseous composition for the same feed and reaction conditions used to make the third product composition. For example, in certain embodiments as otherwise described herein, the reference selectivity for alcohols is no more than 75% of the third selectivity for alcohols (e.g., no more than 60%, or no more than 50%). In certain embodiments as otherwise described herein, the reference selectivity for alcohols is no more than 40% of the third selectivity for alcohols (e.g., no more than 30%, or no more than 25%). In some embodiments as otherwise described herein, the reference selectivity for alcohols is no more than 20% of the third selectivity for alcohols (e.g., no more than 15%, or no more than 10%).

The third product composition can advantageously have a similar selectivity for alcohols compared to the first product composition. Accordingly, in certain embodiments as otherwise described herein, the third selectivity for alcohols is in the range of 50% to 150%, e.g., in the range of 60% to 140%, of the first selectivity for alcohols. For example, in certain embodiments, the third selectivity for alcohols is in the range of 70% to 130%, e.g., in the range of 80% to 120% of the first selectivity for alcohols.

The third product composition can in some embodiments have a decreased selectivity for C₅₊ hydrocarbons. Accordingly, in certain embodiments as otherwise described herein, the selectivity of the third product composition for C₅₊ hydrocarbons is no more than 95%, or no more than 90%. For example, in certain embodiments, the selectivity of the third product composition for C₅₊ hydrocarbons is no more than 85%, or no more than 75%.

In certain embodiments as otherwise described herein, the second selectivity (i.e., of the second product composition) for C₅₊ hydrocarbons is greater than the third selectivity (i.e., of the third product composition) for C₅₊ hydrocarbons. For example, in certain embodiments, the second selectivity for C₅₊ hydrocarbons is at least 105%, e.g., at least 110%, of the third selectivity for C₅₊ hydrocarbons. In particular embodiments, the second selectivity for C₅₊ hydrocarbons is at least 115%, e.g., at least 120% or at least 125%, of the third selectivity for C₅₊ hydrocarbons.

In certain embodiments as otherwise described herein, a reference selectivity for C₅₊ hydrocarbons of the catalyst before the contacting with the second selectivity-modifying gaseous composition for the same feed and reaction conditions used to make the second product composition is greater than the third selectivity for C₅₊ hydrocarbons. For example, in certain embodiments, the reference selectivity for C₅₊ hydrocarbons is at least 105%, e.g., at least 110%, of the third selectivity for C₅₊ hydrocarbons. In particular embodiments, the reference selectivity for C₅₊ hydrocarbons is at least 115%, e.g., at least 120% or at least 125%, of the third selectivity for C₅₊ hydrocarbons.

An advantage of the processes of the present disclosure is that they may be used to alter the product selectivity of a Fischer-Tropsch reaction through only chemical alteration of the catalyst. Accordingly, in certain embodiments as otherwise described herein, the contacting the catalyst with the first selectivity gaseous composition, the contacting the catalyst with the second gaseous feed, the contacting with the second selectivity gaseous composition, and the contacting with the third gaseous feed are performed in a reactor without removing the catalyst therefrom are performed in a reactor without removing the catalyst therefrom.

The second selectivity-modifying gaseous composition is selected to efficiently alter the selectivity of the third product composition resulting from contacted of the third gaseous feed with the catalyst. In certain embodiments as otherwise described herein, the second selectivity-modifying gaseous composition comprises no more than 75 vol% H₂, or no more than 60 vol% H₂. For example, in certain embodiments, the second selectivity-modifying gaseous composition comprises no more than 55 vol% H₂, or no more than 50 vol% H₂, or no more than 45 vol% H₂, or no more than 40 vol% H₂.

In certain embodiments as otherwise described herein, the second selectivity-modifying gaseous composition comprises H₂ and CO in a H₂:CO molar ratio in the range of 0.6:1 to 1.4:1, or in the range of 0.7:1 to 1.3:1, or in the range of 0.8:1 to 1.2:1. For example, the H₂:CO molar ratio may be in the range of 0.9:1 to 1.1:1, or may be approximately 1:1 (e.g., within 5% of 1:1).

In certain embodiments as otherwise described herein, the contacting the catalyst with the second selectivity-modifying gaseous composition occurs as a pressure in the range of 5 barg to 40 barg (e.g., in the range of 10 to 30 barg, or in the range of 15 to 30 barg, or in the range of 20 to 30 barg) and a temperature in the range of 120 °C to 300 °C (e.g., in the range of 130 °C to 280 °C, or in the range of 140 °C to 250 °C).

The contact of the catalyst with the third gaseous feed to provide the third product composition can be performed under any desired set of Fischer-Tropsch reaction conditions. In certain embodiments as otherwise described herein, the contacting the catalyst with the third gaseous feed occurs at a pressure in the range of 150 °C to 300 °C (e.g., in the range of 175 °C to 275 °C, or in the range of 200 °C to 250 °C). In certain embodiments as otherwise described here, the contacting the catalyst with the third gaseous feed occurs at a pressure of 10 barg to 100 barg, or 20 barg to 80 barg.

The person of ordinary skill in the art will select a desirable cobalt-manganese catalyst for use in the processes of the disclosure, based on the disclosure herein. Suitable synthesis catalysts typically may possess a wide variety of transition metal loadings. In certain embodiments as otherwise described herein, the synthesis catalyst comprises at least 0.5 wt% manganese on an elemental basis. In certain embodiments, the synthesis catalyst comprises up to 15 wt% manganese on an elemental basis. For example, the synthesis catalyst may comprise manganese in the range of 0.5 to 15 wt% on an elemental basis, for example, 0.5 to 15 wt%, or 1 to 15 wt%, or 2 to 15 wt%, or 2.5 to 12 wt%, or 3 to 12 wt%, or 4 to 12 wt%, or 5 to 12 wt%, or 2.5 to 11 wt%, or 3 to 11 wt%, or 4 to 11 wt%, or 2.5 to 10 wt%, or 3 to 10 wt%, or 4 to 10 wt%, or 5-14 wt%, or 5-13 wt%, or 5-12 wt%, or 5-11 wt%, or 5-10 wt%. In other embodiments, the catalyst contains no more than 2.5 wt% manganese, or no more than 2 wt% manganese (e.g., no more than 1.5 wt%, or no more than 1 wt% manganese). In certain such embodiments, the catalyst contains at least 0.5 wt% manganese.

In certain embodiments as otherwise described herein, the synthesis catalyst comprises at least 2.5 wt% cobalt on an elemental basis. In certain embodiments, the synthesis catalyst comprises up to 35 wt% cobalt on an elemental basis. For example, in certain embodiments, the synthesis catalyst comprises cobalt in an amount of 2-35 wt%, e.g., 5-35 wt%, or 7-35 wt%, or 10-35 wt%, or 2-25 wt%, or 5-25 wt%, or 7-25 wt%, or 10-25 wt%, on an elemental basis. In certain particular embodiments, the synthesis catalyst comprises cobalt in an amount of 2-20 wt%, e.g., 5-20 wt%, or 7-20 wt%, or 10-20 wt%, or 2-15 wt%, or 5-15 wt%, or 7-15 wt%, an elemental basis.

Without wishing to be bound by theory, it is believed that preparing a catalyst that comprises at least 2.5 wt.% manganese and a manganese to cobalt weight ratio, on an elemental basis, of at least 0.2, by impregnation, the cobalt oxide crystallite sizes in the resulting supported Co-Mn Fischer-Tropsch synthesis catalyst are of a particle size which may give rise to, or contribute to, benefits when the catalyst is utilized in a Fischer-Tropsch reaction. In certain embodiments of the disclosure, the cobalt oxide crystallite (e.g., Co₃O₄) particle sizes resulting from the combination of total amount of manganese and the weight ratio manganese to cobalt weight ratio as described herein are less than 150 Angstroms (15 nm), for example less than 120 Angstroms (12 nm), preferably less than 100 Angstroms (10 nm), such as less than 80 Angstroms (8 nm) or less than 60 Angstroms (6 nm) as defined by X-ray diffraction techniques. Once the Co-Mn Fischer-Tropsch synthesis catalyst is activated and utilized in a Fischer-Tropsch reaction, productivity and selectivity for alcohols can be notably enhanced over cobalt-containing synthesis catalysts comprising no manganese, or an insufficient amount of manganese. Additionally, without being bound by theory, it is believed that the productivity and selectivity for olefins is notably enhanced over cobalt-containing synthesis catalysts comprising no manganese, or an insufficient amount of manganese.

Without being bound by any particular theory, it is believed that the presence of manganese contributes to surface effects on the solid support that influence cobalt oxide crystallite development and dispersivity at the surface. This may derive from the mobility of cobalt-containing precursor compound(s) which are applied to the support material during catalyst preparation, for instance suspended or dissolved in an impregnation solution, whilst in the presence of manganese-containing precursor compound(s). Thus, catalysts especially suitable for use herein can involve cobalt-containing precursor compound(s) and manganese-containing precursor compound(s) being applied to a support material such that they form a mobile admixture at the surface of the support during its preparation.

As described above, inventors have found FT catalysts that comprise mixtures of cobalt and manganese as especially suitable for increasing alcohol production. In certain embodiments as otherwise described herein, the weight ratio of manganese to cobalt in the catalyst is at least 0.05, or at least 0.1, or at least 0.2 on an elemental basis. In particular embodiments, the weight ratio of manganese to cobalt in the catalyst is no more than 4.0, or no more than 3.0, or no more than 2.0 on an elemental basis. In certain embodiments as otherwise described herein, the weight ratio of manganese to cobalt present in the synthesis catalyst is in the range of 0.05 to 3.0 on an elemental basis. For example, in particular embodiments, the weight ratio is in the range of 0.05 to 3, or 0.1 to 3, or 0.2 to 2.5, or 0.2 to 2.0, or 0.05 to 1.75, or 0.0.1 to 1.5, or 0.2 to 1.25, or 0.2 to 1.0, or 0.3 to 1.0. As described herein, this alcohol selectivity can be effectively "switched off' through contact with a first selectivity-modifying gaseous composition.

In certain embodiments as otherwise described herein, the total amount of cobalt and manganese in the synthesis catalyst is no more than 40 wt% on an elemental basis, based on the total weight of the synthesis catalyst. For example, in particular embodiments the total amount of cobalt and manganese in the synthesis catalyst is no more than 30 wt%, or no more than 25 wt%, or no more than 22 wt%, or no more than 20 wt%. In certain embodiments, the total amount of cobalt and manganese in the synthesis catalyst is no more than 15 wt%. In certain embodiments as otherwise described herein, the total amount of cobalt and manganese in the synthesis catalyst is at least 2 wt% on an elemental basis, based on the total weight of the synthesis catalyst. For example, in particular embodiments the total amount of cobalt and manganese in the synthesis catalyst is at least 5 wt%, or at least 8 wt%, or at least 10 wt%.

In certain embodiments, the catalyst used in a process as described herein is a supported Co-Mn Fischer- Tropsch synthesis catalyst comprising cobalt oxide crystallites having a particle size of less than 150 Angstroms (15 nm), preferably less than 100 Angstroms (10 nm), or less than 80 Angstroms (8 nm), and comprising at least 0.5 wt% of manganese, on an elemental basis, based on the total weight of the supported synthesis catalyst; and wherein the weight ratio of manganese to cobalt, on an elemental basis, is 0.05 or greater, and the support material of the supported Co-Mn Fischer-Tropsch synthesis catalyst comprises a material selected from alumina, zirconia, zinc oxide, ceria, and titania. For example, in particular embodiments, the synthesis catalyst comprises a support material that comprises titania, or ceria or is titania or ceria.

The supported Co-Mn Fischer-Tropsch synthesis catalyst used in accordance with the present disclosure may be prepared by any suitable method which is able to provide the required manganese to cobalt weight ratio and the required concentration of manganese on the supported. Preferably, the supported Co-Mn Fischer-Tropsch synthesis catalyst used in accordance with the present disclosure is prepared by a process in which the cobalt and the manganese are impregnated on to the support material.

A suitable impregnation method, for example, comprises impregnating a support material with cobalt-containing compound, which is thermally decomposable to the oxide form, and a manganese-containing compound. Impregnation of the support material with the cobalt-containing compound and the manganese-containing compound may be achieved by any suitable method of which the skilled person is aware, for instance by vacuum impregnation, incipient wetness or immersion in excess liquid.

The incipient wetness technique is so-called because it requires that the volume of impregnating solution be predetermined so as to provide the minimum volume of solution necessary to just wet the entire surface of the support, with no excess liquid. The excess solution technique as the name implies, requires an excess of the impregnating solution, the solvent being thereafter removed, usually by evaporation.

The support material may be in the form of a powder, granulate, shaped particle, such as a preformed sphere or microsphere, or extrudate. Reference herein to a powder or granulate of a support material is understood to refer to free flowing particles of a support material or particles of support material that have undergone granulation and/or sieving to be a particular shape (e.g. spherical) and size range. Reference herein to an "extrudate" is intended to mean a support material that has undergone an extrusion step and therefore may be shaped. In the context of the present disclosure, the powder or granulate is in a form which is suitable for impregnation with a solution of cobalt-containing compound and manganese-containing compound, and subsequent extrusion or forming into other shaped particles.

The support material serves to bind the catalyst particles and may also influence the catalytic activity. In certain embodiments as otherwise described herein, the support material comprises one or more oxide selected from the group consisting of alumina, zirconia, zinc oxide, ceria, and titania. In particular embodiments, the support material is one of alumina, zirconia, zinc oxide, ceria, and titania. For example, in certain embodiments, the catalyst comprises titania (e.g., is titania).

It will be understood that the support material may be in any form provided it is suitable for use as a support for a Fischer-Tropsch synthesis catalyst and also preferably where the support material has not been previously impregnated with sources of metal (i.e., other than cobalt and/or manganese) that may have a deleterious effect on the performance of the active catalyst and may interfere with the benefits of the processes of the disclosure. Thus, whilst support material that has been previously loaded with cobalt and/or manganese metal, or precursors thereof, may be used in accordance with the disclosure, other pre-treatments providing sources of other metals are preferably to be avoided. Preferred support materials are substantially free of extraneous components which might adversely affect the catalytic activity of the system. Thus, preferred support materials are at least 95 % w/w pure, more preferably at least 98 % w/w pure and most preferably at least 99 % w/w pure. Impurities preferably amount to less than 1% w/w, more preferably less than 0.50 % w/w and most preferably less than 0.25 % w/w. The pore volume of the support is preferably more than 0.150ml/g and preferably more than 0.30 ml/g. The average pore radius (prior to impregnation) of the support material is 10 to 500A, preferably 15 to 100A, more preferably 20 to 80 A and most preferably 25 to 60 A. The BET surface area is suitably from 2 to 1000 m²g, preferably from 10 to 600 m²/g, more preferably from 15 to 100 m²/g, and most preferably 30 to 60 m²/g.

The BET surface area, pore volume, pore size distribution and average pore radius may be determined from the nitrogen adsorption isotherm determined at 77K using a Micromeritics TRISTAR 3000 static volumetric adsorption analyser. A procedure which may be used is an application of British Standard methods BS4359:Part 1:1984 'Recommendations for gas adsorption (BET) methods' and BS7591:Part 2:1992, 'Porosity and pore size distribution of materials' - Method of evaluation by gas adsorption. The resulting data may be reduced using the BET method (over the pressure range 0.05-0.20 P/Po) and the Barrett, Joyner & Halenda (BJH) method (for pore diameters of 20-1000 A) to yield the surface area and pore size distribution respectively.

Suitable references for the above data reduction methods are Brunauer, S, Emmett, P H, & Teller, E, J. Amer. Chem. Soc. 60, 309, (1938) and Barrett, E P, Joyner, LG & Halenda P P, J. Am Chem. Soc., 1951 73 373-380.

When in the form of a powder, the median particle size diameter (d50) is preferably less than 50 µm, more preferably less than 25 µm. When the support material is in the form of a granulate, the median particle size diameter (d50) is preferably from 300 to 600 µm. Particle size diameter (d50) may suitably be determined by means of a particle size analyser (e.g. Microtrac S3500 Particle size analyser).

It is known to be beneficial to perform Fischer-Tropsch catalysis with a shaped particle, such as an extrudate, particularly in the case of fixed catalyst bed reactor systems. For instance, it is known that, for a given shape of catalyst particles, a reduction in the size of the catalyst particles in a fixed bed gives rise to a corresponding increase in pressure drop through the bed. Thus, the relatively large shaped particles cause less of a pressure drop through the catalyst bed in the reactor compared to the corresponding powdered or granulated supported catalyst. Shaped particles, such as extrudates, also generally have greater strength and experience less attrition, which is of particular value in fixed bed arrangements where bulk crush strength must be very high.

Reference herein to "impregnation" or "impregnating" is intended to refer to contact of the support material with a solution, or solutions, of, for example, a cobalt-containing compound and a manganese-containing compound, before drying in order to achieve precipitation of the cobalt-containing compound and the manganese-containing compound. Impregnation with a fully dissolved solution, or solutions, of a cobalt-containing compound and a manganese-containing compound ensures good dispersion of the cobalt- containing compound and the manganese-containing compound on the support material and is thus preferred. This is in contrast, for instance, to the use of partially dissolved cobalt- containing compound and/or a partially dissolved manganese-containing compound in 'solid solutions' or suspensions, where the level of dispersion of the cobalt-containing compound and manganese-containing compound across the surface, and in the pores, of the support material can fluctuate depending on the nature of the precipitation on the support material. Furthermore, use of a fully dissolved solution, or solutions, of cobalt-containing compound and manganese-containing compound also has less of an impact upon the resulting morphology and bulk crush strength of an extrudate formed thereafter compared with solid solutions. Nevertheless, benefits of the processes of the present disclosure can also be realised in the case where a solid solution, or solutions, of a partially undissolved cobalt-containing compound and/or manganese-containing compound is used.

Where a powder or granulate of a support material is contacted with a solution, or solutions, of cobalt-containing compound and manganese-containing compound, the amount of solution used preferably corresponds to an amount of liquid which is suitable for achieving a mixture which is of a suitable consistency for further processing, for example for shaping by extrusion. In that case, complete removal of the solvent of the impregnating solution may be effected after formation of the shaped particle, such as an extrudate.

Suitable cobalt-containing compounds are those which are thermally decomposable to an oxide of cobalt following calcination and which are preferably completely soluble in the impregnating solution. Preferred cobalt-containing compounds are the nitrate, acetate or acetylacetonate of cobalt, most preferably the nitrate of cobalt, for example cobalt nitrate hexahydrate. It is preferred to avoid the use of the halides because these have been found to be detrimental.

Suitable manganese-containing compounds are those which are thermally decomposable following calcination and which are preferably completely soluble in the impregnating solution. Preferred manganese-containing compounds are the nitrate, acetate or acetylacetonate of manganese, most preferably the acetate of manganese.

The solvent of the impregnating solution(s) may be either an aqueous solvent or a non-aqueous, organic solvent. Suitable non-aqueous organic solvents include, for example, alcohols (e.g. methanol, ethanol and/or propanol), ketones (e.g. acetone), liquid paraffinic hydrocarbons and ethers. Alternatively, aqueous organic solvents, for example an aqueous alcoholic solvent, may be employed. Preferably, the solvent of the impregnating solution(s) is an aqueous solvent.

In preferred embodiments, the impregnation of the support material with a cobalt-containing compound and a manganese-containing compound occurs in a single step, without any intermediate drying or calcination steps to separate the loading of the different components. As the skilled person will appreciate, the cobalt-containing compound and manganese-containing compound may be applied to the support material successively or simultaneously in separate impregnation solutions or suspensions, or preferably an impregnation solution or suspension comprising both the cobalt-containing compound and the manganese-containing compound is used.

The concentration of the cobalt-containing compound and the manganese-containing compound, in the impregnating solution(s) is not particularly limited, although preferably the cobalt-containing compound and the manganese-containing compound are fully dissolved, as discussed hereinbefore. When a powder or granulate of support material is impregnated and immediately followed by an extrusion step, the amount of the impregnating solution(s) is preferably suitable for forming an extrudable paste.

In a preferred embodiment, the concentration of the impregnating solution is sufficient to afford a supported catalyst containing from 5 wt% to 35 wt% of cobalt, more preferably from 7.5 wt% to 25 wt% of cobalt, even more preferably from 10 to 20 wt% of cobalt, on an elemental basis, based on the total weight of the supported synthesis catalyst.

In another preferred embodiment, the concentration of the impregnating solution is sufficient to afford a supported catalyst containing from 0.5 wt% to 15 wt% of manganese, preferably from 1.0 wt% to 12.5 wt% of manganese, for example from 1.0 to 10 wt% of manganese, or even 1.0 to 8.0 wt% of manganese, on an elemental basis, based on the total weight of the supported synthesis catalyst, following drying and calcination.

A suitable concentration of cobalt-containing compound and/or manganese-containing compound is, for example, 0.1 to 15 moles/litre.

It will be appreciated that where the support material is in powder or granulate form, once impregnated with a cobalt containing compound and a manganese-containing compound, the impregnated support material may be extruded or formed into shaped particles at any suitable stage before or after drying and calcining.

Impregnation of the support material is usually followed by drying of the impregnating solution in order to effect precipitation of the cobalt-containing compound and the manganese-containing compound on to the support material and preferably also to remove bound solvent of the impregnating solution (e.g. water). Drying therefore does not, for instance, lead to full decomposition of the cobalt-containing compound or otherwise lead to a change in oxidation state of the cobalt-containing compound. As will be appreciated, in embodiments where an extrusion is performed, complete drying and removal of solvent (e.g. bound solvent) of the impregnating solution may occur after forming of a shaped particle, for example by extrusion. Drying is suitably conducted at temperatures from 50 °C to 150 °C, preferably 75 °C to 125 °C. Suitable drying times are, for example, from 5 minutes to 72 hours. Drying may suitably be conducted in a drying oven or in a box furnace, for example, under the flow of an inert gas at elevated temperature.

Where a shaped particle, such as an extrudate, is impregnated, it will be appreciated that the support may be contacted with the impregnating solution by any suitable means including, for instance, vacuum impregnation, incipient wetness or immersion in excess liquid, as mentioned hereinbefore. Where a powder or granulate of support material is impregnated, the powder or granulate may be admixed with the impregnating solution by any suitable means of which the skilled person is aware, such as by adding the powder or granulate to a container of the impregnating solution and stirring.

Where a step of forming a shaped particle, such as an extrusion step, immediately follows impregnation of a powder or granulate, the mixture of powder or granulate and impregnating solution may be further processed if it is not already in a form which is suitable for forming a shaped particle, for instance by extrusion. For instance, the mixture may be mulled to reduce the presence of larger particles that may not be readily extruded or otherwise formed into a shaped particle, or the presence of which would otherwise compromise the physical properties of the resulting shaped particle, for example an extrudate. Mulling typically involves forming a paste which is suitable for shaping, such as by extrusion. Any suitable mulling or kneading apparatus of which the skilled person is aware may be used for mulling in the context of the present disclosure. For example, a pestle and mortar may suitably be used in some applications or a Simpson muller may suitably be employed. Mulling is typically undertaken for a period of from 3 to 90 minutes, preferably for a period of 5 minutes to 30 minutes. Mulling may suitably be undertaken over a range of temperatures, including ambient temperatures. A preferred temperature range for mulling is from 15 °C to 50 °C. Mulling may suitably be undertaken at ambient pressures. As stated hereinbefore, it will be appreciated that complete removal of bound solvent from the impregnation solution may be conducted to effect complete precipitation after forming of the shaped particle, such as by extrusion.

In embodiments where a calcination step is performed on an impregnated powder or granulate, thereby completely removing solvent of the impregnation solution, the calcined powder or granulate may also be further processed in order to form a mixture which is suitable for forming into shaped particles, for example by extruding. For instance, an extrudable paste may be formed by combining the calcined powder or granulate with a suitable solvent, for example a solvent used for impregnation, preferably an aqueous solvent, and mulled as described above.

Preparation of the supported Co-Mn Fischer-Tropsch synthesis catalyst involves a calcination step. As will be understood, calcination is required for converting the cobalt-containing compound which has been impregnated on the support material into an oxide of cobalt. Thus, calcination leads to thermal decomposition of the cobalt-containing compound, and not merely removal of bound solvent of an impregnating solution, as for instance in the case of drying.

Calcination may be performed by any method known to those of skill in the art, for instance in a fluidized bed or rotary kiln at a temperature of at least 250 °C, preferably from 275 °C to 500 °C. In some embodiments, calcination may be conducted as part of an integrated process where calcination and reductive activation of the synthesis catalyst to yield a reduced Fisher-Tropsch synthesis catalyst are performed in the same reactor. In a particularly preferred embodiment, the supported Co-Mn Fischer-Tropsch synthesis catalyst used in the process of the disclosure is obtained or obtainable from a process comprising the steps of:
(a) impregnating a support material with: a cobalt-containing compound and a manganese-containing compound in a single impregnation step to form an impregnated support material; and
(b) drying and calcining the impregnated support material to form the supported Co-Mn Fischer-Tropsch synthesis catalyst.

A particular advantage of this embodiment is the expediency with which a support material may be modified and converted into a supported Co-Mn Fischer-Tropsch synthesis catalyst using only a single impregnation step followed by a drying and calcination step. Thus, in preferred embodiments, the support material used in connection with the processes of the disclosure has undergone no prior modification, for instance by the addition of promoters, dispersion aids, strength aids and/or binders, or precursors thereof, before impregnation in step (a) of the process.

The supported Co-Mn Fischer-Tropsch synthesis catalyst used in the process of the present disclosure may additionally comprise one or more promoters, dispersion aids or binders. Promoters may be added to promote reduction of an oxide of cobalt to cobalt metal, preferably at lower temperatures. Preferably, the one or more promoters is selected from the list consisting of ruthenium, palladium, platinum, rhodium, rhenium, chromium, nickel, iron, molybdenum, tungsten, zirconium, gallium, thorium, lanthanum, cerium and mixtures thereof. Promoter is typically used in a cobalt to promoter atomic ratio of up to 250:1 and more preferably up to 125:1, still more preferably up to 25:1, and most preferably 10:1. In preferred embodiments, the one or more promoters are present in the cobalt- containing Fischer-Tropsch synthesis catalyst obtained in an amount from 0.1 wt% to 3 wt%, on an elemental basis, based on the total weight of the supported synthesis catalyst.

The addition of the promoters, dispersion aids, strength aids, or binders may be integrated at several stages of the catalyst preparation process. Preferably, the promoters, dispersion aids or binders, or precursors thereof, is/are introduced during impregnation step(s) where the cobalt-containing compound and manganese-containing compound are introduced. The supported Co-Mn Fischer-Tropsch synthesis catalyst may conveniently be converted into a reduced supported Co-Mn Fischer-Tropsch synthesis catalyst by reductive activation by any known means of which the skilled person is aware which is capable of converting cobalt oxide to the active cobalt metal. Thus, in one embodiment, the process of the disclosure further comprises a preceding step of reducing a Co-Mn Fischer-Tropsch synthesis catalyst to form a reduced Co-Mn Fischer-Tropsch synthesis catalyst by contacting with a hydrogen-containing gas stream. The step of forming a reduced synthesis catalyst may be carried out batch wise or continuously in a fixed bed, fluidised bed or slurry phase reactor, or in-situ in the same reactor as will be subsequently used for the Fischer-Tropsch synthesis reaction. Reduction is suitably performed at a temperature of from 150 °C to 500 °C, preferably from 200 °C to 400 °C, more preferably from 250 °C to 350 °c.

As will be appreciated, the gaseous reactant mixture supplied to the Fischer-Tropsch reaction may also be suitable for reducing the supported Co-Mn Fischer-Tropsch synthesis catalyst to form a reduced supported Co-Mn Fischer-Tropsch synthesis catalyst in situ, without requiring any preceding or distinct reductive activation step.

In the Fischer-Tropsch reaction of the disclosure, the volume ratio of hydrogen to carbon monoxide (H₂:CO) in the gaseous reactant mixture is at least 1 : 1, preferably at least 1.1 : 1, more preferably at least 1.2 : 1, more preferably at least 1.3 : 1, more preferably at least 1.4 : 1, more preferably at least 1.5 : 1, or even at least 1.6 : 1. In some or all embodiments of the present disclosure, the volume ratio of hydrogen to carbon monoxide (H₂:CO) in the gaseous reactant mixture is at most 5 : 1, preferably at most 3 : 1, most preferably at most 2.2 : 1. Examples of suitable volume ratios of hydrogen to carbon monoxide (H₂:CO) in the gaseous reactant mixture include the ranges: from 1 : 1 to 5 : 1; from 1.1 : 1 to 3 : 1 ; from 1.2 : 1 to 3 : 1; from 1.3 : 1 to 2.2 : 1 ; from 1.4 : 1 to 5 : 1 ; from 1.4 : 1 to 3 : 1 ; from 1.4 : 1 to 2.2 : 1 ; from 1.5 : 1 to 3 : 1 ; from 1.5 : 1 to 2.2 : 1 ; and, from 1.6:1 to 2.2:1. The gaseous reactant stream may also comprise other gaseous components, such as nitrogen, carbon dioxide, water, methane and other saturated and/or unsaturated light hydrocarbons, each preferably being present at a concentration of less than 30% by volume.

Conventional Fischer-Tropsch temperatures may be used in order to prepare the product compositions in accordance with the present disclosure. For example, the temperature of the contacting of a mixture of hydrogen and carbon monoxide gases (e.g., in the form of a synthesis gas mixture) with a supported cobalt-manganese Fischer-Tropsch catalyst may suitable be in the range from 100 to 400 °C, such as from 100 to 350 °C, or 100 to 300 °C, or 100 to 250 °C, or 150 to 400 °C, or 150 to 350 °C, or 150 to 300 °C, or 150 to 250 °C. In certain embodiments, the contacting is conducted at a temperature of no more than 350 °C, e.g., no more than 325 °C, or no more than 300 °C, or no more than 280 °C, or no more than 260 °C. The pressure of the contacting (i.e., the temperature of the Fischer-Tropsch reaction) can in certain embodiments suitably be in the range from 10 to 100 bara (from 1 to 10 MPa), such as from 15 to 75 bara (from 1.5 to 7.5 MPa), or from 20 to 50 bara (from 2.0 to 5.0 MPa). For example, in certain embodiments the contacting is conducted at a pressure of no more than 7.5 MPa absolute.

In particular embodiments, the temperature of the Fischer-Tropsch reaction is in the range from 150 to 350 °C, more preferably from 180 to 300 °C, and most preferably from 200 to 260 °C. In preferred embodiments, the pressure of the Fischer-Tropsch reaction is in the range from 10 to 100 bar (from 1 to 10 MPa), more preferably from 10 to 60 bar (from 1 to 6 MPa) and most preferably from 20 to 45 bar (from 2 to 4.5 MPa).

The Fischer-Tropsch synthesis reaction may be performed in any suitable type of reactor, for example it may be performed in a fixed bed reactor, a slurry bed reactor, or a CANs reactor.

In another aspect of the disclosure, there is provided a supported Co-Mn Fischer-Tropsch synthesis catalyst comprising at least 0.5 wt% of manganese, on an elemental basis, based on the total weight of the supported synthesis catalyst; and wherein the weight ratio of manganese to cobalt present, on an elemental basis, is 0.05 or greater, the support material of the supported Co-Mn Fischer-Tropsch synthesis catalyst comprises a material selected from titania, zinc oxide, zirconia, and ceria, and wherein the supported Co-Mn Fischer-Tropsch synthesis is prepared by impregnation.

For the purposes of this disclosure, Co₃O₄ crystallite particle sizes are determined by X-ray diffraction.

As will be appreciated, the support material and methods for preparing the supported Co-Mn Fischer-Tropsch synthesis catalysts of the above further aspects of the disclosure may be as defined hereinbefore. For example, the synthesis catalysts are preferably obtained or obtainable from a process comprising the steps of:
(a) impregnating a support material with: a cobalt-containing compound and a manganese-containing compound in a single impregnation step to form an impregnated support material; and
(b) drying and calcining the impregnated support material to form the supported Co-Mn Fischer-Tropsch synthesis catalyst.

The supported Co-Mn Fischer-Tropsch synthesis catalysts of the above further aspects of the disclosure may also be used for i) increasing the selectivity of a Fischer-Tropsch process for the production of alcohols; and/or ii) increasing conversion in a Fischer-Tropsch process.

In a yet further aspect of the disclosure, there is provided a method for controlling cobalt oxide crystallite size in the preparation of a supported cobalt-containing Fischer- Tropsch synthesis catalyst, said method comprising the step of supplying acetic acid, or a metal salt of acetic acid, during the impregnation of a support material with a cobalt- containing compound, wherein the metal is selected from the group consisting of ruthenium, palladium, platinum, rhodium, rhenium, manganese, chromium, nickel, iron, molybdenum, tungsten, zirconium, gallium, thorium, lanthanum, cerium and mixtures thereof; preferably wherein the metal is selected from manganese, ruthenium, rhenium and platinum, more preferably the metal is manganese.

The processes of the disclosure will now be further described by reference to the following Examples which are illustrative only. In the Examples, CO conversion is defined as moles of CO used/moles of CO fed x 100 and carbon selectivity as moles of CO attributed to a particular product/moles of CO converted x 100. Unless otherwise stated, temperatures referred to in the Examples are applied temperatures and not catalyst/bed temperatures. Unless otherwise stated, pressures referred to in the Examples are absolute pressures.

### EXAMPLES

The Examples that follow are illustrative of specific embodiments of the methods of the disclosure, and various uses thereof. They are set forth for explanatory purposes only, and are not to be taken as limiting the scope of the disclosure.

### Example 1:

Experiments were conducted on a variety of catalyst compositions support at a pressure of 30 barg. Syntheses were performed before treatment with the first selectivity-modifying gaseous composition to generate the first product composition, and after treatment to determine the second product composition. The first selectivity-modifying composition was pure H₂, and was allowed to contact the catalyst at 200-250 °C and at a pressure of 30 barg for 24 hours. The catalysts were activated at 300 °C for 15hrs in 100%H2 at atmospheric pressure. The testing conditions were at 30barg, 1.8H2:CO with online GC product analysis. The results are shown below, in Table 1:

**Table 1**

| | **Catalyst % Co:** | **10%** | | | | | | | **20%** | |
|---|---|---|---|---|---|---|---|---|---|---|
| | **Catalyst % Mn:** | **0%** | **1%** | **2%** | **3%** | **5%** | **7.5%** | **10%** | **4%** | **5%** |
| **CO Conversion** | **1^{st} Product Composition** | 39.0 | 43.5 | 40.4 | 43.5 | 38.4 | 37.5 | 38.3 | 45.0 | 39.3 |
| | **2^{nd} Product Composition** | 49.5 | 51.3 | 61.3 | 64.5 | 62.3 | 64.4 | 66.9 | 65.4 | 59.6 |
| | | | | | | | | | | |
| **Total Alcohol Selectivity** | **1^{st} Product Composition** | 0.8 | 1.7 | 7.8 | 12.6 | 14.4 | 13.3 | 13.5 | 12.8 | 11.8 |
| | **2^{nd} Composition** | 0.7 | 1.0 | 0.7 | 0.5 | 0.6 | 0.5 | 0.6 | 0.3 | 0.4 |
| | | | | | | | | | | |
| **Hexanol selectivity** | **1^{st} Product Composition** | 0.1 | 0.1 | 1.1 | 1.5 | 1.8 | 1.8 | 1.8 | 1.6 | 1.4 |
| | **2^{nd} Product Composition** | 0.1 | 0.1 | 0.1 | 0.0 | 0.0 | 0.0 | 0.0 | 0.0 | 0.0 |
| | | | | | | | | | | |
| **1-Pentanol Selectivity** | **1^{st} Product Composition** | 0.1 | 0.2 | 1.1 | 1.8 | 2.0 | 1.8 | 1.9 | 1.7 | 1.4 |
| | **2^{nd} Product Composition** | 0.1 | 0.1 | 0.1 | 0.0 | 0.1 | 0.0 | 0.0 | 0.0 | 0.0 |
| | | | | | | | | | | |
| **Total OH + O** | **1^{st} Product Composition** | 5.1 | 6.6 | 22.1 | 30.5 | 30.8 | 35.4 | 32.7 | 25.0 | 21.3 |
| | **2^{nd} Product Composition** | 4.9 | 7.5 | 5.0 | 4.1 | 4.9 | 5.1 | 4.9 | 3.0 | 3.3 |
| | | | | | | | | | | |
| **C₅₊ Selectivity** | **1^{st} Product Composition** | 88.6 | 89.1 | 76.2 | 72.4 | 67.6 | 60.3 | 60.5 | 65.6 | 72.3 |
| | **2^{nd} Product Composition** | 90.7 | 91.4 | 93.0 | 94.1 | 93.4 | 92.0 | 92.1 | 94.1 | 93.9 |
| | | | | | | | | | | |
| | **Temperature (°C)** | 197.7 | 196.8 | 200. 8 | 201. 7 | 203. 0 | 209.7 | 212.0 | 204.9 | 196.8 |

Accordingly, treatment of the catalyst with the first selectivity gaseous mixture surprisingly decreases the alcohol selectivity and increases the C₅₊ selectivity. The above results are summarized in Table 2:

**Table 2**

| **Catalyst % Co:** | **10%** | | | | | | | **20%** | |
|---|---|---|---|---|---|---|---|---|---|
| **Catalyst % Mn:** | **0%** | **1%** | **2%** | **3%** | **5%** | **7.5%** | **10%** | **4%** | **5%** |
| **Change in Alcohol Selectivity after Treatment** | -0.1 | -0.7 | -7.1 | -12.1 | -13.8 | -12.8 | -12.9 | -12.5 | -11.4 |
| **Change in C₅₊ Hydrocarbon Selectivity after Treatment** | +2.1 | +2.3 | +16.8 | +21.7 | +25.8 | +31.5 | +31.5 | +28.5 | +21.6 |

Treatment with the first selectivity-modifying gaseous mixture is an effective way to alter the selectivity of the Fischer-Tropsch reaction. The decrease in alcohol selectivity and increase in C₅₊ hydrocarbon selectivity is observed throughout catalyst manganese loadings, although it is more dramatic for catalysts with greater than 1% manganese. However, useful changes are still observed with low manganese catalysts.

Table 3, highlighting the increased selectivity for alcohols following a carbon monoxide rich gas feed, and the switching of that selectivity following a hydrogen rich feed

| **Catalyst Loading** | **Exp. No** | **C₅₊ selectivity** | **Alcohol Selectivity** |
|---|---|---|---|
| **Description** | | **%** | **(C₁₋₈) mol%** |
| **10%Co/1%Mn/TiO2** | **Baseline** | **91.2** | **0.8** |
| | **After a high CO Treatment** | **69.0** | **6.3** |
| | **After a high H2 Treatment** | **92.5** | **0.6** |
| **10%Co/2%Mn/TiO2** | **Baseline** | **89.1** | **1.1** |
| | **After a high CO Treatment** | **30.0** | **23.7** |
| | **After a high H2 Treatment** | **90.1** | **0.7** |
| **10%Co/3%Mn/TiO2** | **Baseline** | **63.8** | **7.3** |
| | **After a high CO Treatment** | **33.0** | **21.5** |
| | **After a high H2 Treatment** | **74.7** | **2.8** |
| **10%Co/5%Mn/TiO2** | **Baseline** | **65.0** | **15.5** |
| | **After a high CO Treatment** | **64.4** | **13.0** |
| | **After a high H2 Treatment** | **68.6** | **4.2** |

Various exemplary embodiments of the disclosure include, but are not limited to the enumerated embodiments listed below, which can be combined in any number and in any combination that is not technically or logically inconsistent.

The particulars shown herein are by way of example and for purposes of illustrative discussion of certain embodiments of the present disclosure only and are presented in the cause of providing what is believed to be the most useful and readily understood description of the principles and conceptual aspects of various embodiments of the disclosure. In this regard, no attempt is made to show details associated with the methods of the disclosure in more detail than is necessary for the fundamental understanding of the methods described herein, the description taken with the examples making apparent to those skilled in the art how the several forms of the methods of the disclosure may be embodied in practice. Thus, before the disclosed processes and devices are described, it is to be understood that the aspects described herein are not limited to specific embodiments, apparatus, or configurations, and as such can, of course, vary. It is also to be understood that the terminology used herein is for the purpose of describing particular aspects only and, unless specifically defined herein, is not intended to be limiting.

The terms "a," "an," "the" and similar referents used in the context of describing the methods of the disclosure (especially in the context of the following embodiments and claims) are to be construed to cover both the singular and the plural, unless otherwise indicated herein or clearly contradicted by context.

All methods described herein can be performed in any suitable order of steps unless otherwise indicated herein or otherwise clearly contradicted by context. The use of any and all examples, or exemplary language (e.g., "such as") provided herein is intended merely to better illuminate the methods of the disclosure and does not pose a limitation on the scope of the disclosure. No language in the specification should be construed as indicating any non-claimed element essential to the practice of the methods of the disclosure.

Unless the context clearly requires otherwise, throughout the description and the claims, the words 'comprise', 'comprising', and the like are to be construed in an inclusive sense as opposed to an exclusive or exhaustive sense; that is to say, in the sense of "including, but not limited to". Words using the singular or plural number also include the plural and singular number, respectively. Additionally, the words "herein," "above," and "below" and words of similar import, when used in this application, shall refer to this application as a whole and not to any particular portions of the application.

As will be understood by one of ordinary skill in the art, each embodiment disclosed herein can comprise, consist essentially of or consist of its particular stated element, step, ingredient or component. As used herein, the transition term "comprise" or "comprises" means includes, but is not limited to, and allows for the inclusion of unspecified elements, steps, ingredients, or components, even in major amounts. The transitional phrase "consisting of' excludes any element, step, ingredient or component not specified. The transition phrase "consisting essentially of' limits the scope of the embodiment to the specified elements, steps, ingredients or components and to those that do not materially affect the embodiment.

All percentages, ratios and proportions herein are by weight, unless otherwise specified.

Notwithstanding that the numerical ranges and parameters setting forth the broad scope of the disclosure are approximations, the numerical values set forth in the specific examples are reported as precisely as possible. Any numerical value, however, inherently contains certain errors necessarily resulting from the standard deviation found in their respective testing measurements.

Groupings of alternative elements or embodiments of the disclosure are not to be construed as limitations. Each group member may be referred to and claimed individually or in any combination with other members of the group or other elements found herein. It is anticipated that one or more members of a group may be included in, or deleted from, a group for reasons of convenience and/or patentability. When any such inclusion or deletion occurs, the specification is deemed to contain the group as modified thus fulfilling the written description of all Markush groups used in the appended claims.

Some embodiments of various aspects of the disclosure are described herein, including the best mode known to the inventors for carrying out the methods described herein. Of course, variations on these described embodiments will become apparent to those of ordinary skill in the art upon reading the foregoing description. The skilled artisan will employ such variations as appropriate, and as such the methods of the disclosure can be practiced otherwise than specifically described herein. Accordingly, the scope of the disclosure includes all modifications and equivalents of the subject matter recited in the claims appended hereto as permitted by applicable law. Moreover, any combination of the above-described elements in all possible variations thereof is encompassed by the disclosure unless otherwise indicated herein or otherwise clearly contradicted by context.

Various exemplary embodiments of the disclosure include, but are not limited to the enumerated embodiments listed here, which can be combined in any number and in any combination that is not technically or logically inconsistent.

The particulars shown herein are by way of example and for purposes of illustrative discussion of certain embodiments of the present disclosure only and are presented in the cause of providing what is believed to be the most useful and readily understood description of the principles and conceptual aspects of various embodiments of the disclosure. In this regard, no attempt is made to show details associated with the methods of the disclosure in more detail than is necessary for the fundamental understanding of the methods described herein, the description taken with the examples making apparent to those skilled in the art how the several forms of the methods of the disclosure may be embodied in practice. Thus, before the disclosed processes and devices are described, it is to be understood that the aspects described herein are not limited to specific embodiments, apparatus, or configurations, and as such can, of course, vary. It is also to be understood that the terminology used herein is for the purpose of describing particular aspects only and, unless specifically defined herein, is not intended to be limiting.

The terms "a," "an," "the" and similar referents used in the context of describing the methods of the disclosure (especially in the context of the following embodiments and claims) are to be construed to cover both the singular and the plural, unless otherwise indicated herein or clearly contradicted by context.

All methods described herein can be performed in any suitable order of steps unless otherwise indicated herein or otherwise clearly contradicted by context. The use of any and all examples, or exemplary language (e.g., "such as") provided herein is intended merely to better illuminate the methods of the disclosure and does not pose a limitation on the scope of the disclosure. No language in the specification should be construed as indicating any non-claimed element essential to the practice of the methods of the disclosure.

Unless the context clearly requires otherwise, throughout the description and the claims, the words 'comprise', 'comprising', and the like are to be construed in an inclusive sense as opposed to an exclusive or exhaustive sense; that is to say, in the sense of "including, but not limited to". Words using the singular or plural number also include the plural and singular number, respectively. Additionally, the words "herein," "above," and "below" and words of similar import, when used in this application, shall refer to this application as a whole and not to any particular portions of the application.

As will be understood by one of ordinary skill in the art, each embodiment disclosed herein can comprise, consist essentially of or consist of its particular stated element, step, ingredient or component. As used herein, the transition term "comprise" or "comprises" means includes, but is not limited to, and allows for the inclusion of unspecified elements, steps, ingredients, or components, even in major amounts. The transitional phrase "consisting of' excludes any element, step, ingredient or component not specified. The transition phrase "consisting essentially of" limits the scope of the embodiment to the specified elements, steps, ingredients or components and to those that do not materially affect the embodiment.

All percentages, ratios and proportions herein are by weight, unless otherwise specified.

Notwithstanding that the numerical ranges and parameters setting forth the broad scope of the disclosure are approximations, the numerical values set forth in the specific examples are reported as precisely as possible. Any numerical value, however, inherently contains certain errors necessarily resulting from the standard deviation found in their respective testing measurements.

Groupings of alternative elements or embodiments of the disclosure are not to be construed as limitations. Each group member may be referred to and claimed individually or in any combination with other members of the group or other elements found herein. It is anticipated that one or more members of a group may be included in, or deleted from, a group for reasons of convenience and/or patentability. When any such inclusion or deletion occurs, the specification is deemed to contain the group as modified thus fulfilling the written description of all Markush groups used in the appended claims.

Some embodiments of various aspects of the disclosure are described herein, including the best mode known to the inventors for carrying out the methods described herein. Of course, variations on these described embodiments will become apparent to those of ordinary skill in the art upon reading the foregoing description. The skilled artisan will employ such variations as appropriate, and as such the methods of the disclosure can be practiced otherwise than specifically described herein. Accordingly, the scope of the disclosure includes all modifications and equivalents of the subject matter recited in the claims appended hereto as permitted by applicable law. Moreover, any combination of the above-described elements in all possible variations thereof is encompassed by the disclosure unless otherwise indicated herein or otherwise clearly contradicted by context.

The phrase "at least a portion" as used herein is used to signify that, at least, a fractional amount is required, up to the entire possible amount.

In closing, it is to be understood that the various embodiments herein are illustrative of the methods of the disclosures. Other modifications that may be employed are within the scope of the disclosure. Thus, by way of example, but not of limitation, alternative configurations of the methods may be utilized in accordance with the teachings herein. Accordingly, the methods of the present disclosure are not limited to that precisely as shown and described.

## Claims

1. A process for converting a mixture of hydrogen and carbon monoxide gases to a product composition comprising alcohols and liquid hydrocarbons via Fischer-Tropsch synthesis in the presence of a supported cobalt-manganese Fischer-Tropsch synthesis catalyst, the process comprising:
optionally, contacting the catalyst with a first gaseous feed comprising carbon monoxide and hydrogen for at least 12 hours to provide via Fischer-Tropsch synthesis a first product composition comprising C₅₊ hydrocarbons and one or more alcohols with a first selectivity for alcohols and a first selectivity for C5+ hydrocarbons; then
contacting the catalyst with a selectivity-modifying gaseous composition comprising at least 35 vol% H₂ and a H₂:CO molar ratio of at least 2.0 at a pressure in the range of 10 barg to 40 barg and a temperature in the range of 150 °C to 300 °C; and then
contacting the catalyst with a second gaseous feed comprising carbon monoxide and hydrogen to provide a second product composition comprising C₅₊ hydrocarbons, with a second selectivity for alcohols of no more than 5%, and/or a second selectivity for C₅₊ hydrocarbons of at least 80%.

2. The process of any of claim 1, further comprising, before the contacting with the first gaseous feed and/or the first selectivity-modifying gaseous composition, contacting the catalyst with an activation gaseous composition comprising at least 50 vol% H₂ at a pressure in the range of 2 barg to 30 barg and a temperature in the range of 250 °C to 450 °C.

3. The process of any of claims 1-2, wherein the first selectivity-modifying gaseous composition comprises at least 50 vol% H₂ (e.g., at least 60 vol% H₂, or at least 70 vol% H₂, or at least 80 vol% H₂, or at least 90 vol% H₂, or at least 95 vol% H₂, or at least 99 vol% H₂).

4. The process of any of claims 1-3, wherein the first selectivity-modifying gaseous composition has a H₂:CO molar ratio of at least 3 (e.g., at least 4, or at least 5, or at least 7, or at least 10).

5. The process of any of claims 1-4, wherein the contacting of the catalyst with the first selectivity-modifying gaseous composition is at a pressure in the range of 5 barg to 35 barg (e.g., in the range of 20 to 32 barg, or at approximately 30 barg).

6. The process of any of claims 1-5, wherein the contacting of the catalyst with the first selectivity-modifying gaseous composition is at a temperature in the range of 150 °C to 275 °C (e.g., in the range of 200 °C to 250 °C).

7. The process of any of claims 1-6, wherein the second selectivity for alcohols is no more than 20%, e.g., no more than 15%, or no more than 10%, of the first selectivity for alcohols.

8. The process of any of claims 1-7, wherein the contacting with the first gaseous feed (when performed), the contacting with the first selectivity-modifying gaseous composition, and the contacting with the second gaseous feed are performed in a reactor without removing the catalyst therefrom.

9. The process of any of claims 1-8, further comprising:
monitoring the second selectivity for alcohols and/or the second selectivity for C₅₊ hydrocarbons;
determining whether the second selectivity for alcohols is greater than an alcohols threshold value, and/or whether the second selectivity for C₅₊ hydrocarbons is less than a hydrocarbons threshold value; and
if the second selectivity for alcohols is greater than the alcohols threshold value, and/or if the second selectivity for C₅₊ hydrocarbons selectivity is less than the hydrocarbons threshold value, contacting the catalyst with the first selectivity gaseous composition.

10. A process for converting a mixture of hydrogen and carbon monoxide gases to a product composition comprising alcohols and liquid hydrocarbons via Fischer-Tropsch synthesis in the presence of a supported cobalt-manganese Fischer-Tropsch synthesis catalyst, the process comprising:
optionally, contacting the catalyst with a first gaseous feed comprising carbon monoxide and hydrogen for at least 12 hours to provide via Fischer-Tropsch synthesis a first product composition comprising C₅₊ hydrocarbons and one or more alcohols with a first selectivity for alcohols and a first selectivity for C5+ hydrocarbons; then
contacting the catalyst with a selectivity-modifying gaseous composition comprising H₂ and CO in a ratio in the range of pure carbon monoxide to a H2:CO ratio of 1.5:1 at a pressure in the range of 5 barg to 40 barg and at a temperature in the range of 100 °C and 300 °C; and then
contacting the catalyst with a second gaseous feed comprising carbon monoxide and hydrogen to provide a third product composition comprising C₅₊ hydrocarbons and alcohol, with a selectivity of greater than 5% for alcohols, and/or a selectivity of no more than 92% for C₅₊ hydrocarbons.

11. The process of claim 10, wherein the contacting of the catalyst with the third gaseous feed is performed with a third selectivity for alcohols of at least 10%, e.g., at least 12%.

12. The process of any of claims 10-11, wherein the second selectivity-modifying gaseous composition comprises no more than 75 vol% H₂ (e.g., no more than 60 vol% H₂, or no more than 55 vol% H₂, no more than 50 vol% H₂, or no more than 45 vol% H₂, or no more than 40 vol% H₂), and/or a H₂:CO molar ratio in the range of 0.25:1 to 0.4:1 (e.g., in the range of 0.7:1 to 1.3:1, or in the range of 0.8:1 to 1.2:1, or in the range of 0.9:1 to 1.1:1, or approximately 1:1).

13. The process of any of claims 10-12, wherein contacting the catalyst with the second selectivity-modifying gaseous composition at a pressure in the range of 5 barg to 35 barg (e.g., in the range of 6 to 16 barg, or in the range of 7 to 14 barg, or in the range of 8 to 12 barg) and a temperature in the range of 120 °C to 250 °C (e.g., in the range of 130 °C to 220 °C, or in the range of 140 °C to 200 °C).

14. The process of any of claims 10-13, wherein the contacting the catalyst with the third gaseous feed occurs at a temperature in the range of 150 °C to 300 °C (e.g., in the range of 175 °C to 275 °C, or in the range of 200 °C to 250 °C), and at a pressure of 10 barg to 100 barg (e.g., in the range of 20 barg to 80 barg).

15. The process of any of claims 1-14, wherein the catalyst comprises cobalt in an amount of 2-35 wt%, e.g., 5-35 wt%, or 7-35 wt%, or 10-35 wt%, or 2-25 wt%, or 5-25 wt%, or 7-25 wt%, or 10-25 wt%, on an elemental basis.

16. The process of any of claims 1-15, wherein the catalyst comprises manganese in an amount of 0.5-20 wt%, e.g., 1-20 wt%, or 2-20 wt%, or 10-20 wt%, or 2-15 wt%, or 5-15 wt%, or 7-15 wt%, an elemental basis.

17. The process of any of claims 1-16, wherein the catalyst comprises a support material that comprises at least one oxide selected from alumina, silica, zirconia, zinc oxide, ceria, and titania.

18. The process of any of claims 1-17, wherein the catalyst comprises titania, wherein a weight ratio of manganese to cobalt in the catalyst is at least 0.05 on an elemental basis.

19. The process of any of claims 1-18, wherein the weight ratio of manganese to cobalt in the catalyst is in the range of 0.05 to 3.0 on an elemental basis (e.g., in the range 0.05 to 3, or 0.1 to 3, or 0.33 to 3, or 0.2 to 2.5, or 0.2 to 2.0, or 0.2 to 1.75, or 0.2 to 1.5, or 0.2 to 1.25, or 0.2 to 1.0, or 0.3 to 1.0).

20. The process of any of claims 1-19, wherein the catalyst comprises up to 15 wt%, e.g., in the range of 0.5-15 wt% manganese on an elemental basis (e.g., in the range of 1-14 wt%, or 2-13 wt%, or 5-12 wt%, or 5-11 wt%, or 3-10 wt%.
